Europäisches Patentamt

(19) European Patent Office

Office européen des brevets

(11) Veröffentlichungsnummer: **0 082 218**
**B1**

(12) **EUROPÄISCHE PATENTSCHRIFT**

(45) Veröffentlichungstag der Patentschrift:
09.04.86

(21) Anmeldenummer: 81110745.7

(22) Anmeldetag: 23.12.81

(51) Int. Cl.⁴: **A 61 B 17/06**

(54) **Trägervorrichtung für chirurgisches Nahtmaterial.**

(43) Veröffentlichungstag der Anmeldung:
29.06.83 Patentblatt 83/26

(45) Bekanntmachung des Hinweises auf die Patenterteilung:
09.04.86 Patentblatt 86/15

(84) Benannte Vertragsstaaten:
AT BE CH DE FR GB IT LI LU NL SE

(56) Entgegenhaltungen:
CH - A - 344 183
CH - A - 448 376
DE - A - 2 618 662
DE - C - 2 700 077
US - A - 2 499 890
US - A - 4 287 987

(73) Patentinhaber: INTERMEDICAT GMBH,
Gerliswilstrasse 43, CH-6020 Emmenbrücke (CH)

(72) Erfinder: Ploch, Horst, Am Sportplatz 1,
D-3509 Elfershausen (DE)
Erfinder: Heinemann, Dieter, Birkenweg 6,
D-3508 Melsungen-Röhrenfurth (DE)
Erfinder: Mell, Gerhard, Guntershäuser Strasse 28,
D-3501 Fuldabrück (DE)

(74) Vertreter: von Kreisler, Alek, Dipl.-Chem. et al,
Deichmannhaus am Hauptbahnhof, D-5000 Köln 1 (DE)

## Beschreibung

Die Erfindung betrifft eine Trägervorrichtung für chirurgisches Nahtmaterial, bestehend aus einem Trägerkörper in Form einer flachen Scheibe, deren umlaufende Randfläche eine Wickelfläche zum Aufwickeln eines Fadens des Nahtmaterials bildet.

Eine bekannte Trägervorrichtung dieser Art (DE-C-27 00 077) weist ein Faltblatt auf, auf dem ein Trägerkörper in Form einer flachen ringförmigen Scheibe zum Aufspulen des Fadens und ein Blöckchen aus weichelastischem Material zum Einstechen der mit dem Faden fest verbundenen Nadel angeordnet sind. Die Trägervorrichtung ist im zusammengeklappten Zustand der Faltkarte in einer sterilen Umhüllung eingepackt. Nach dem Entnehmen aus der Umhüllung wird die Faltkarte aufgeklappt, der Faden von der Umfangsfläche des Trägerkörpers abgespult und schließlich die Nadel aus dem Blöckchen herausgezogen. Ein Nachteil dieser bekannten Trägervorrichtung besteht darin, daß zur Entnahme des Nahtmaterials zunächst die Faltkarte aus der sterilen Folienverpackung herausgenommen und anschließend die Faltkarte geöffnet werden muß. Dies erfordert eine Reihe von Handgriffen, die bei einer Operation störend sein können. Ein weiterer Nachteil besteht darin, daß das Nahtmaterial vor dem Öffnen der sterilen Folienverpackung nicht sichtbar und nicht erkennbar ist, weil es sich im Innern der gefalteten Faltkarte befindet.

Ferner sind Trägervorrichtungen für chirurgisches Nahtmaterial bekannt, bei denen der Trägerkörper eine flache Karte ist, auf die der Faden aufgewickelt ist (DE-GM 71 22 075) bzw. solche Trägervorrichtungen, bei denen der Trägerkörper eine Faltkarte ist, in die einige vorgewickelte Windungen des Fadens eingeschoben sind (US-PS 3 319 782). Die Trägerkörper weisen Einbuchtungen oder Einschnitte zum Festlegen eines Fadenendes oder einer Nadel auf. Wenn die Nadel an dem Karton einer Karte befestigt ist, kann sie nur gegen einen gewissen Widerstand aus dem Karton herausgezogen werden. Dadurch entsteht die Gefahr des Abgleitens oder der Mitnahme von Partikeln des Materials des Trägerkörpers. Bei einem auf eine Karte aufgewickelten Faden muß der Faden zunächst von der Karte abgewickelt werden, bevor er zum Nähen benutzt wird. Dies hat den Nachteil, daß sich Nadel und Faden während der Abnahme von dem Trägerkörper verwirren können und daß sich eine Schlinge bzw. später ein Knoten im Faden bildet. Vielfach weist ein von derartigen Kartonträgern entnommener Faden Knickstellen oder Verformungen auf, die sogar die Reißfestigkeit des Fadens herabsetzen können.

Der Erfindung liegt die Aufgabe zugrunde, eine Trägervorrichtung der eingangs genannten Art zu schaffen, die besonders einfach in ihrem Aufbau und übersichtlich in der Handhabung ist und sowohl beim Aufwickeln des Fadens in der Fabrik als auch beim Abwickeln des Fadens durch den Anwender eine einfache, übersichtliche und sichere Handhabung ermöglicht.

Zur Lösung dieser Aufgabe ist erfindungsgemäß vorgesehen, daß der Trägerkörper an oder in der Nähe der Randfläche zum Festlegen eines oder mehrerer Fadenenden und/oder einer bzw. mehrerer Nadeln geeignet ausgebildet ist.

Bei der erfindungsgemäßen Trägervorrichtung wird der Faden des Nahtmaterials auf den Umfangsrand einer Scheibe gewickelt, so daß die Flachseiten der Scheibe vom Faden freigehalten werden. Damit werden Knickbildungen des Faden durch Vermeidung großer Umlenkwinkel ausgeschaltet. Der Faden kann durch Ziehen an seinem einen Ende kontinuierlich von dem Trägerkörper abgezogen werden, ohne daß Festklemmungen oder Schlingenbildungen eintreten. Wichtig ist, daß die Wickelfläche des Trägerkörpers keine scharfkantigen Winkel im Bereich von 90° oder mehr aufweist, an denen beim Abziehen des Fadens durch Überlagern mehrerer Fadenwindungen Verklemmungen auftreten können.

Der Trägerkörper, auf dessen Umfangsfläche der Faden aufgewickelt wird, dient gleichzeitig zur Fixierung des Fadenendes bzw. einer oder mehrerer Nadeln. Es ist daher nicht erforderlich, den Trägerkörper seinerseits auf einer Karte o.dgl. anzubringen, sondern der Trägerkörper bildet das einzige Teil der Trägervorrichtung. Seine Flachseiten können für die Anbringung von Aufdrucken, z.B. Benutzungshinweisen oder Angaben über die Art des Nahtmaterials, benutzt werden. Diese Flachseiten werden von dem Nahtmaterial nicht einmal teilweise bedeckt, so daß sie für eine Bedruckung voll zur Verfügung stehen.

Gemäß einer bevorzugten Ausführungsform der Erfindung besteht der Trägerkörper aus einem Polymerschaumstoff. Dieser Schaumstoff sollte so weich sein, daß die Nadel ohne größeren Kraftaufwand und ohne Beschädigung derselben eingestochen und wieder herausgezogen werden kann. Er sollte ferner eine gewisse Elastizität besitzen, so daß eine eingestochene Nadelspitze von ihm umschlossen und festgehalten wird. Schaumstoffe die diese Anforderungen erfüllen, sind unter den Bezeichnungen Novopolen bzw. Alvedit bekannt.

Der Trägerkörper kann aus einem offenporigen oder geschlossenporigen Schaumstoff bestehen. Die Verwendung eines elastischen Schaumstoffs hat außerdem den Vorteil, daß der Faden mit einer sehr geringen Spannung um den Trägerkörper herumgewickelt werden kann, ohne daß die Gefahr des unkontrollierten Herabfallens der gesamten Fadenwicklung besteht. Diese Eigenschaft kann noch dadurch verbessert werden, daß das Schaumstoffmaterial längs der Wickelfläche offenzellig ist, so daß der Faden an den Zellenwänden durch leichtes Verhaken seiner Fasern einen gewissen Halt erhält.

Gemäß einer bevorzugten Ausführungsform der Erfindung ist die Randfläche aus zwei im wesentlichen parallelen Seitenwänden des Trägerkörpers und mindestens einer runden Stirnwand gebildet. Die zweite Stirnwand des Trägerkörpers kann zwei jeweils unter einem Winkel von etwa 30° bis 60° zu en Seitenwänden verlaufende geradlinige Abschnitte aufweisen. Diese geradlinigen Abschnitte dienen zum Einstechen und somit zum vorübergehenden Fixieren der Nadel des Nahtmaterials. Die Nadel ist somit leicht zugänglich und leicht auffindbar, ohne in störender Weise den in der Verpackungshülle für die Trägervorrichtung vorzusehenden Raum zu vergrößern.

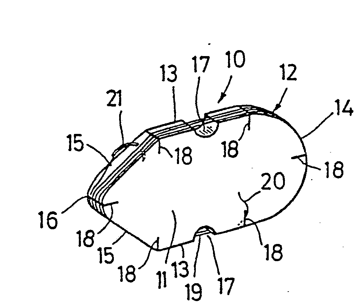

Im Folgenden wird unter Bezugnahme auf die einzige Figur der Zeichnung ein bevorzugtes Ausführungsbeispiel der Erfindung näher erläutert.

Die Zeichnung zeigt eine perspektivische Ansicht eines Trägerkörpers zusammen mit dem auf ihn aufgewickelten chirurgischen Nahtmaterial.

Der dargestellte Trägerkörper 10 besteht aus einer Scheibe aus einem Schaumstoff mit durchgehend konstanter Stärke von etwa 4 mm. Die Hauptflächen 11 dieser Scheibe sind eben und an ihnen sind die Schaumstoffporen geschlossen. Die umlaufende Randfläche 12 verläuft rechtwinklig zu den Hauptflächen 11 und in ihr sind die Schaumstoffporen offen. Der Trägerkörper 10 kann durch Ausschneiden oder Ausstanzen aus einem bahnoder plattenförmigen Schaumstoffmaterial hergestellt sein.

Die Randfläche 12 besteht aus zwei parallelen Seitenflächen 13, die an ihren einen Enden durch eine kreisbogenförmige Stirnwand 14 und an ihren anderen Enden durch zwei geradlinige Abschnitte 15 miteinander verbunden sind. Die geradlinigen Abschnitte 15 verlaufen jeweils unter einem Winkel von etwa 40° zu der Längsmittelachse des Trägerkörpers (und damit auch zu den Seitenwänden 13) und sie vereinigen sich in einer abgerundeten Spitze 16.

Um den Trägerkörper während des Aufwickelns des chirurgischen Nahtmaterials in einer entsprechenden Vorrichtung festzuhalten, sind im Bereich der Seitenwände 13 Ausnehmungen 17 vorgesehen, in die entsprechende Greifwerkzeuge der Maschine eingreifen können, ohne den Aufwickelvorgang zu behindern. Nach Fertigstellung der Wicklung können die Greifwerkzeuge unter dem Faden aus den Ausnehmungen 17 herausgezogen werden.

Bei dem beschriebenen Ausführungsbeispiel beträgt der gegenseitige Abstand der Seitenwände 13 26 mm und der Krümmungsradius der Stirnwand 14 beträgt 13 mm. Der Radius der Abrundung der Spitze 16 beträgt 6 mm.

Die Randfläche 12 des Trägerkörpers 10 ist im vorliegenden Fall ebenflächig und rechtwinklig zu den Hauptflächen 11. Sie kann aber auch rinnenformig ausgebildet sein, um ein seitliches Abspringen des Fadens zusätzlich zu erschweren.

Der Trägerkörper 10 weist mehrere von der Randfläche 12 ausgehende Schlitze 18 auf, in denen das Ende eines Fadens 19 festgelegt werden kann. Ein derartiges fixiertes Fadenende ist in der Zeichnung mit 20 bezeichnet. Der Faden 19 ist - ausgehend von dem fixierten Fadenende 20 - in mehreren Windungen auf die Randfläche 12 aufgewickelt. An seinem entgegengesetzten Ende ist die Nadel 21 befestigt, die in einem der Abschnitte 15 in die Randfläche 12 eingestochen ist.

Die schrägen Abschnitte 15 der Randfläche 12 haben den Vorteil, daß innerhalb einer gedachten rechteckigen Umhüllungslinie des Trägerkörpers 10 alle vorkommenden Nadelgrößen mit ihren entsprechenden Maßen untergebracht werden können. Der Trägerkörper 10 eignet sich daher für Nahtmaterial mit unterschiedlichen Fadenlängen und Nadelgrößen gleichermaßen. Da der Faden 19 auf die Umfangsfläche 12 aufgewickelt ist und somit keine scharfkantigen Umlenkungen und keine Umlenkungen um einen Winkel,der größer ist als etwa 100°,erleidet, ist er durch einfaches Abziehen, d.h. durch Ziehen an der Nadel 21, verfügbar. Es ist also nicht erforderlich, den Faden 19 vor Gebrauch zunächst vollständig abzuwickeln. Zu berücksichtigen ist hierbei, daß auch das Fadenende 20, das in einem der Schlitze 18 festgelegt ist, wegen der Weichheit des Schaumstoffmaterials mit relativ geringem Kraftaufwand aus dem Schlitz 18 herausgezogen werden kann.

Für die Verbraucherinformation relevante Produktangaben,wie Fadenmaterial, Länge, Pharmakopoebezeichnung, Nadeldimensionen usw.,werden vorteilhafterweise direkt auf eine oder beide Hauptflächen 11 des Trägerkörpers 10 aufgebracht. Hierdurch kann auf die sonst in die Packung eingelegten Informationsträger in Form separater Zettel usw. verzichtet werden. Diese Informationen können auch auf ein Klebeetikett oder eine Folie aufgedruckt werden, die anschließend auf die Hauptflächen 11 aufgeklebt wird.

Der Nahtmaterialträger 10 mit dem auf seine Randfläche 12 aufgewickelten Nahtmaterial 19,21 wird in einer (nicht dargestellten) rechteckigen durchsichtigen Verpackung keimdicht untergebracht. Vor Benutzung des Nahtmaterials wird die Verpackung geöffnet und der Nahtmaterialträger 10 mit dem Nahtmaterial herausgenommen. Anschließend wird der Nahtmaterialträger 10 mit Daumen und Zeigefinger an den Hauptflächen 11 angefaßt und das Nahtmaterial kann durch Ziehen an der Nadel 21 abgezogen werden. Anstelle eines Fadens mit an einem Ende fest angebrachter Nadel 21 kann auch ein Faden ohne Nadel auf den Nahtmaterialträger aufgewickelt werden, oder ein Faden der an jedem seiner Enden eine Nadel aufweist.

## Patentansprüche

1. Trägervorrichtung für chirurgisches Nahtmaterial, bestehend aus einem Trägerkörper (10) in Form einer flachen Scheibe, deren umlaufende Randfläche (12) eine Wickelfläche zum Aufwickeln eines Fadens des Nahtmaterials bildet, dadurch gekennzeichnet, daß der Trägerkörper (10) an oder in der Nähe der Randfläche (12) zum Festlegen eines oder mehrerer Fadenenden und/oder einer bzw. mehrerer Nadeln (21) geeignet ausgebildet ist.

2. Trägervorrichtung nach Anspruch 1, dadurch gekennzeichnet, daß der Trägerkörper (10) aus einem Polymerschaumstoff besteht.

3. Trägervorrichtung nach Anspruch 1 oder 2, dadurch gekennzeichnet, daß die Randfläche (12) aus zwei im wesentlichen parallelen Seitenwänden (13) des Trägerkörpers (10) und mindestens einer runden Stirnwand (14) gebildet ist.

4. Trägervorrichtung nach Anspruch 3, dadurch gekennzeichnet, daß die zweite Stirnwand des Trägerkörpers (10) zwei jeweils unter einem Winkel von etwa 30° bis 60° zu den Seitenwänden (13) verlaufende geradlinige Abschnitte (15) aufweist.

5. Trägervorrichtung nach einem der Ansprüche 1 bis 4, dadurch gekennzeichnet, daß der Trägerkörper (10) mindestens einen Querschlitz (18) zum Festhalten eines Fadens (19) aufweist.

6. Trägervorrichtung nach einem der vorhergehenden Ansprüche, dadurch gekennzeichnet, daß in der Randfläche (12) zwei einander gegenüberliegende Ausnehmungen (17) angeordnet sind.

7. Trägervorrichtung nach einem der vorhergehenden Ansprüche, dadurch gekennzeichnet, daß die Randfläche (12) rinnenförmig ausgebildet ist.

8. Trägervorrichtung nach einem der Ansprüche 2 bis 7, dadurch gekennzeichnet, daß der Schaumstoff an der Randfläche (12) offenzellig ist.

## Claims

1. Holder for a surgical suture material, comprising a support body (10) shaped as a flat plate whose circumjacent marginal face (12) forms a winding surface to wind up a thread the suture material, characterized in that at the marginal face (12) or in the vicinity thereof, the support body (10) is of a design adequate to fix one or more threadnends and/or one or more needles (21).

2. Holder according to claim 1, characterized in that the support body (10) is made of a polymer foam material.

3. Holder according to claim 1 or 2, characterized in that the marginal face (12) is formed of at least two substantially parallel side walls (13) of the support body (12) and of at least one round end wall (14).

4. Holder according to claim 3 characterized in that the second end wall of the support body (10) comprises two rectilinear sections (15) extending each at an angle of about 30° to 60° relative to the side walls (13).

5. Holder according to one of claims 1 to 4, characterized in that the support body (10) comprises at least one transverse slot (18) for fixing a thread (19).

6. Holder according to one of the preceding claims, characterized in that the marginal face (12) includes two opposite recesses (17).

7. Holder according to one of the preceding claims, characterized in that the marginal face (12) is groove-shaped.

8. Holder according to one of claims 2 to 7, characterized in that the foam material is open-celled at the marginal face (12).

## Revendications

1. Support pour sutures chirurgicales constitué d'un corps (10) formé d'une plaquette dont la surface périphérique (12) forme une surface d'enroulement d'un fil de suture, caractérisé en ce que le corps (10), à sa surface périphérique (12) ou à proximité de celle-ci, est agencé de manière appropriée à la fixation d'une ou de plusieurs extrémités de fil et/ou d'une ou de plusieurs aiguilles (21).

2. Support selon la revendication 1, caractérisé en ce que le corps (10) est en matière alvéolaire à base de polymères.

3. Support selon l'une des revendications 1 ou 2, caractérisé en ce que la surface périphérique (12) est formée de deux parois latérales sensiblement parallèles (13) du corps (10) et d'au moins une paroi frontale ronde (14).

4. Support selon la revendication 3, caractérisé en ce que la deuxième paroi frontale du corps (10) présente deux parties rectilignes (15) faisant chacune un angle d'environ 30 à 60° avec les parois latérales (13).

5. Support selon l'une quelconque des revendications 1 à 4, caractérisé en ce que le corps (10) présente au moins une fente transversale (18) pour la fixation d'un fil (19).

6. Support selon l'une quelconque des revendications 1 à 5, caractérisé en ce que deux évidements opposés (17) sont réalisés dans la surface périphérique (12).

7. Support selon l'une quelconque des revendications 1 à 6, caractérisé en ce que la surface périphérique (12) est en forme de gouttière.

8. Support selon l'une quelconque des revendications 2 à 7, caractérisé en ce qu'à la surface périphérique (12), la matière alvéolaire est à alvéoles ouverts.